# EUROPEAN PATENT APPLICATION

(11) **EP 1 640 459 A1**
(43) Date of publication of application: **29.03.2006**
(21) Application number: 03736026.0
(22) Date of filing: 04.06.2003
(51) Int. Cl.: C12Q 1/48, G01N 33/40, G01N 33/14, C12N 15/00

(54) **METHOD OF DIAGNOSING CANCER AND METHOD OF JUDGING CANCER RISK**

(71) Applicant: Hokkaido Technology Licensing Office Co., Ltd., Sapporo-Shi, Hokkaido 060-0807 (JP)
(72) Inventor: SAKATA, Koh-ichi, Sapporo-shi, Hokkaido 003-0022 (JP); SOMEYA, Masanori, Sapporo-shi, Hokkaido 064-0807 (JP); MATSUMOTO, Yoshihisa, Tokyo 113-0031 (JP)
(74) Representative: Curtis, Philip Anthony
(86) International application number: PCT/JP2003/007079
(87) International publication number: WO 2004/108953

(57) **Abstract**

The method for diagnosing cancer according to the present invention is performed by measuring DNA-dependent protein kinase activity in cells derived from a test subject. With the method for diagnosing cancer according to the present invention, the presence of cancer cells can be investigated, whatever the organ or the cause of the carcinogenesis is. Themethodfor determining cancer susceptibility according to the present invention is performed by measuring DNA-dependent protein kinase activity in cells derived from a test subject.

SEQUENCE LISTING

<110> Hokkaido Technology Licensing Office Co. Ltd.
<120> Method for diagnosis of cancer
<130> PCTF157
<140>
<141>
<160> 1
<170> PatentIn Ver. 2.1
<210> 1
<211> 15
<212> PRT
<213> Homo sapiens

<400> 1
Glu Pro Pro Leu Ser Gln Glu Ala Phe Ala Asp Leu Trp Lys Lys
1               5                   10                  15

## Description

### Field of the Invention

The present invention relates to methods for diagnosing cancer and methods for determining cancer susceptibility. Specifically, the present invention relates to methods that can diagnose cancer and cancer susceptibility by measuring DNA-dependent protein kinase activity.

### Description of the Related Art

Methods for diagnosing human cancer using a biological sample, such as serum, are known. For example, methods for diagnosing cancer by measuring a tumor marker in a biological sample such as serum have been developed. With regard to the tumor marker, for example, prostatic specific antigen (PSA) which is a marker of prostatic cancer, squamous cell carcinoma related antigen (SCC) which is a marker of cervical carcinoma, alpha-fetoprotein (AFP) which is a marker of liver cancer, and carcinoembryonic antigen (CEA) which is a marker of colon cancer are known. With regard to high sensitive methods for measuring the tumor marker, for example, a radioimmunoassay (RIA), an enzyme immunoassay (EIA), and a fluorescence immunoassay (FIA), in which different monoclonal antibodies against the tumor marker are used, have been developed.

Conventional tumor markers are targeted to diagnose cancer of a particular organ, and every tumor marker can be used to diagnose cancer of only a particular organ. Additionally, some organs do not have a proper cancer marker. Therefore, the conventional tumor markers cannot be widely applied to the diagnosis of cancer in general. Furthermore, the conventional tumor markers are not cancer-specific substances in a precise sense. Namely, the tumor markers are also produced in a normal living body at some level. Therefore, these markers are difficult to be used in the determination of early stage of cancer when the production level of the tumor marker is low and are difficult to be used in the determination of cancer susceptibility which means a tendency to suffer from cancer.

Therefore, it is an object of the present invention to provide a method for investigating the presence of cancer cells whatever the organ or the cause of the carcinogenesis is. Specifically, it is an object of the present invention to provide a method for diagnosing cancer and a method for determining cancer susceptibility.

### Detailed Description of the Invention

The inventors of the present invention have studied intensively to accomplish the above-mentioned object and, as a result, have found that the above-mentioned object can be achieved by measuring activity of DNA-dependent protein kinase that is an enzyme playing an important role in repair of double-strand DNA break. Thus, the present invention has been completed.

The present invention has been completed on the basis of the above-mentioned finding and provides a method for diagnosing cancer by measuring DNA-dependent protein kinase activity in cells derived from a test subject.

The present invention further provides a method for diagnosing cancer including the steps of measuring DNA-dependent protein kinase activity in cells derived from a test subject; measuring DNA-dependent protein kinase activity in cells derived from a healthy subject; and comparing the DNA-dependent protein kinase activity in cells derived from the test subject and the DNA-dependent protein kinase activity in cells derived from the healthy subject.

The present invention further provides a cancer diagnosis kit for diagnosing cancer by the above-mentioned method for diagnosing cancer. The cancer diagnosis kit includes at least a peptide substrate that is phosphorylated by DNA-dependent protein kinase.

The present invention further provides a method for determining cancer susceptibility by measuring DNA-dependent protein kinase activity in cells derived from a test subject.

The present invention further provides a method for determining cancer susceptibility including the steps of measuring DNA-dependent protein kinase activity in cells derived from a test subject; measuring DNA-dependent protein kinase activity in cells derived from a healthy subject; and comparing the DNA-dependent protein kinase activity in cells derived from the test subject and the DNA-dependent protein kinase activity in cells derived from the healthy subject.

The present invention further provides a cancer-susceptibility determination kit for determining cancer susceptibility by the above-mentioned method for determining cancer susceptibility. The cancer-susceptibility determination kit includes at least a peptide substrate that is phosphorylated by DNA-dependent protein kinase.

### Brief Description of the Drawings

FIG. 1 is a graph showing relationship between DNA-dependent protein kinase activity and chromosome abnormality.
FIG. 2 is a graph showing results of measurement of DNA-dependent proteinkinase activity in lymphoid cells of cancer patients and a normal group.

### Best Mode for Carrying Out the Invention

A method for diagnosing cancer according to the present invention will now be described.

The method for diagnosing cancer according to the present invention is performed by measuring DNA-dependent protein kinase activity in cells derived from a test subject. Specifically, the method for diagnosing cancer according to the present invention includes the steps of measuring DNA-dependent protein kinase activity in cells derived from a test subject; measuring DNA-dependent protein kinase activity in cells derived from a healthy subject; and comparing the DNA-dependent protein kinase activity in cells derived from the test subject and the DNA-dependent protein kinase activity in cells derived from the healthy subject.

Genes (DNA molecules) in vivo receive various damages such as intrastrand cross-link, nucleotide modification, nucleotide excision, and duplex intrastrand cross-link from the environment. These damages are main causes of mutation. The accumulation of mutation causes malignant transformation of cells; thus, the mutation is deeply involved in malignant transformation. Among the above-mentioned damages, double-stranded DNA break is the most serious DNA damage.

The whole picture of proteins involved in a repair mechanism and a repair process of the double-stranded DNA break has being clarified. The outline of the repair mechanism will now be described. Ku-subunit of DNA-dependent protein kinase binds to broken ends of the double-stranded DNA, and recruits catalytic subunit (DNA-PKcs). The activated DNA-dependent protein kinase phosphorylates, for example, XRCC4 protein binding to DNA ligase IV. With this, activated or localized DNA ligase IV rejoins the double strand break of DNA. DNA-dependent protein kinase is an enzyme playing an important role in the repair process of double-stranded DNA break.

It has been found that cancer diagnosis is possible by measuring activity of such DNA-dependent protein kinase.

Then, a method for measuring DNA-dependent protein kinase activity in cells derived from a test subject will be described.

Examples of the cells used in the measurement of DNA-dependent protein kinase activity include lymphoid cells and fibroblasts . Lymphoid cells are preferable.

The measurement of DNA-dependent protein kinase activity (phosphorylation activity) using lymphoid cells will now be described.

Lymphoid cells can be obtained from blood by specific gravity centrifugation. Specifically, lymphoid cells can be obtained by layering the blood on Lymphoprep (manufactured by Nycomed) and centrifuging it to isolate a lymphoid cell fraction.

The resulting lymphoid cells are disrupted and protein content in the cells is measured. The lymphoid cells are disrupted by, but not limited to this method to disrupt cells, freezing and then thawing. The lymphoid cells can be disrupted by repeating this process three times.

Then, the disrupted lymphoid cells are reacted with a peptide substrate (for example, a peptide including an amino acid sequence of a part of human p53 suppressor protein) that is phosphorylated by DNA-dependent protein kinase in a reaction buffer (containing ³²P-ATP) under the presence or absence of DNA; thus, phosphorylation reaction is performed. Examples of the reaction buffer for the phosphorylation reaction include phosphate-buffered saline and HEPES buffer, and pH of the buffer is at or near the optimal pH for DNA-dependent protein kinase.

After the completion of the phosphorylation reaction, the reaction solution is spotted on filter paper. After the washing of the filter paper, the filter paper is dried with ethanol. The remaining radioactivity on the filter paper is measured with a liquid scintillation counter. The radioactivity per unit protein is calculated as the DNA-dependent protein kinase activity.

DNA-dependent protein kinase activity in cells derived from a healthy subject is measured by the same manner as the above.

Then, cancer diagnosis is performed by comparing the DNA-dependent protein kinase activity in cells derived from the test subject and the DNA-dependent protein kinase activity in cells derived from the healthy subject.

Specifically, when the DNA-dependent protein kinase activity of the test subject is lower than that in cells derived from the healthy subject, the possibility that the test subject is suffering with cancer is high. Reversely, when the activity of the test subject is higher than that of the healthy subject, the possibility that the test subject is not suffering with cancer is high.

The method for measuring DNA-dependent protein kinase activity is described hereinbefore, but the present invention is not limited to this. Anymethod for measuring DNA-dependent protein kinase activity is within the scope of the present invention.

It is thought that DNA-dependent protein kinase activity is decreased in all kinds of cancer. Therefore, the method for diagnosing cancer according to the present invention can be used for diagnosis of all kinds of cancer. Examples of such cancer include breast cancer, uterine cancer, head and neck cancer, malignant lymphoma, lung cancer, esophageal cancer, colon cancer, and pancreatic cancer.

A cancer diagnosis kit for diagnosing cancer by the method according to the present invention will now be described. The cancer diagnosis kit according to the present invention is a kit for diagnosing cancer by the above-mentioned method for diagnosing cancer of the present invention and hence includes at least a peptide substrate that is phosphorylated by DNA-dependent protein kinase. Examples of the peptide substrate to be phosphorylated by DNA-dependent protein kinase include peptides containing an amino acid sequence that is a part of human p53 suppressor protein or XRCC4 protein. An example of the peptide substrate is shown as SEQ ID NO:1.

The cancer diagnosis kit according to the present invention may further include a buffer for the reaction. Examples of such a buffer include the buffers exemplified in the above-mentioned method for diagnosing cancer, and the buffer preferably includes ³²P-ATP for labeling the substrate in order to detect the phosphorylation by DNA-dependent protein kinase. The cancer diagnosis kit according to the present invention can be used for performing the above-mentioned method for diagnosing cancer of the present invention and is effective for performing the cancer diagnosis.

Then, a method for determining cancer susceptibility according to the present invention will be described.

In the method for determining cancer susceptibility according to the present invention, DNA-dependent protein kinase activity in cells derived from a test subject is measured. Specifically, the method for diagnosing cancer according to the present invention includes the steps of measuring DNA-dependent protein kinase activity in cells derived from a test subject; measuring DNA-dependent protein kinase activity in cells derived from a healthy subject; and comparing the DNA-dependent protein kinase activity in cells derived from the test subject and the DNA-dependent protein kinase activity in cells derived from the healthy subject.

In the method for determining cancer susceptibility according to the present invention, the method for measuring DNA-dependent protein kinase activity is the same as that described in the above-mentioned method for diagnosing cancer of the present invention.

Namely, cancer susceptibility is determined by comparing DNA-dependent protein kinase activity in cells derived from a test subject and DNA-dependent protein kinase activity in cells derived from a healthy subject.

Specifically, when the DNA-dependent protein kinase activity in cells derived from the test subject is lower than that in cells derived from the healthy subject, it is determined that the cells of the test subject are prone to develop cancer. Reversely, when the activity of the test subject is higher than that of the healthy subject, it is determined that the cells of the test subject hardly develop cancer.

According to the method for determining cancer susceptibility of the present invention, DNA-dependent protein kinase activity in cells derived from the test subject is measured. Then, when DNA-dependent protein kinase activity is low, it is expected that mutation is prone to occur and cancer is prone to develop. Therefore, the method can be used for selecting subjects to be examined for cancer screening more selectively from healthy subjects.

As mentioned above, it is thought that DNA-dependent protein kinase activity is decreased in all kinds of cancer. Therefore, the method for determining cancer susceptibility according to the present invention can be used for diagnosis of all kinds of cancer. Examples of such cancer include cancer described in the above-mentioned method for diagnosing cancer of the present invention.

Then, cancer susceptibility determination kit for determining cancer susceptibility by the method for determining cancer susceptibility according to the present invention will be described. The cancer susceptibility determination kit according to the present invention is for determining cancer susceptibility by the above-mentioned method for determining cancer susceptibility and includes at least a peptide substrate phosphorylated by DNA-dependent protein kinase. Examples of the peptide substrate phosphorylated by DNA-dependent protein kinase include the peptide substrates that are described in the above-mentioned cancer diagnosis kit.

The cancer susceptibility determination kit may further include a buffer for the reaction. Examples of the buffer include the buffers exemplified in the above-mentioned method for diagnosing cancer, and the buffer preferably includes ³²P-ATP for labeling the substrate in order to detect the phosphorylation by DNA-dependent protein kinase. The cancer susceptibility determination kit according to the present invention can be used for performing the above-mentioned method for determining cancer susceptibility of the present invention and is effective for performing the cancer susceptibility determination.

### Examples

The following Examples are given for the purpose of illustration only and are not intended to limit the scope of the present invention.

### Example 1

### Relationship between DNA-dependent protein kinase activity and chromosome abnormality

Relationship between DNA-dependent protein kinase activities in lymphoid cells of a normal group or cancer patients and chromosome abnormalities of the respective cells was investigated. The lymphoid cells of the cancer patients were derived from patients suffering from breast cancer, uterine cancer, head and neck cancer, or malignant lymphoma.

The measurement of DNA-dependent protein kinase activity in the lymphoid cells was performed as follows:
Lymphoid cells were obtained from blood of the normal group and the cancer patients. Each 20 mL blood of the healthy subjects and the cancer patients was layered on Lymphoprep (manufactured by Nycomed) and was centrifuged at 1500 rpm at 4°C for 30 min. Then, a portion including lymphoid cells was collected to obtain the lymphoid cells. The lymphoid cells thus obtained were frozen and then thawed. This process was repeated three times to disrupt the lymphoid cells. Then, the amount of protein of the disrupted lymphoid cells was measured.

A reaction buffer (pH 7.2, HEPES-NaOH containing 100 pmole ³²P-ATP and 990 pmole ATP) containing a peptide substrate (shown as SEQ ID NO: 1) that is phosphorylated by DNA-dependent protein kinase was added to the disrupted lymphoid cells at a ratio of 5 µg of the peptide substrate per 1.25 µg of the protein obtained from the lymphoid cells in the above. Then, 0.4 ng DNA was added to the reaction solution mixture to phosphorylate the peptide substrate. As a control, the peptide substrate was added to the discrupted lymphoid cells without the addition of the DNA.

The phosphorylation reaction was conducted at 37° C for 10 min. After the termination of the phosphorylation reaction, the reaction solution was spotted on filter paper. After the washing of the filter paper, the filter paper was dried with ethanol. The radioactivity on the filter paper was measured with a liquid scintillation counter. The control value was subtracted from the actually measured value to obtain the observed value.

Chromosome abnormality was measure as follows:
Each 2 mL blood was collected from 10 healthy subjects (the normal group) and 10 cancer patients who were used in the above. The collected blood was added to 10 mL culture solution (RPMI-1640, manufactured by Sigma) containing 2 mL fetal bovine serum, and 100 µL phytohemaglutinin (PHA, manufactured by Murex) and 40 µL Colcemid (manufactured by Gibco) were added to the resulting mixture. The mixture was cultured under 5% CO₂ at 37° C for 48 hr. After the culture, the cells were fixed with methanol/acetate acid and were mounted onto a glass slide. Then, chromosome abnormality in the lymphoid cells was observed by Giemsa stain. Two hundred cells were counted for every sample and abnormality frequency was indicated by the number of chromosome segments per 100 cells.

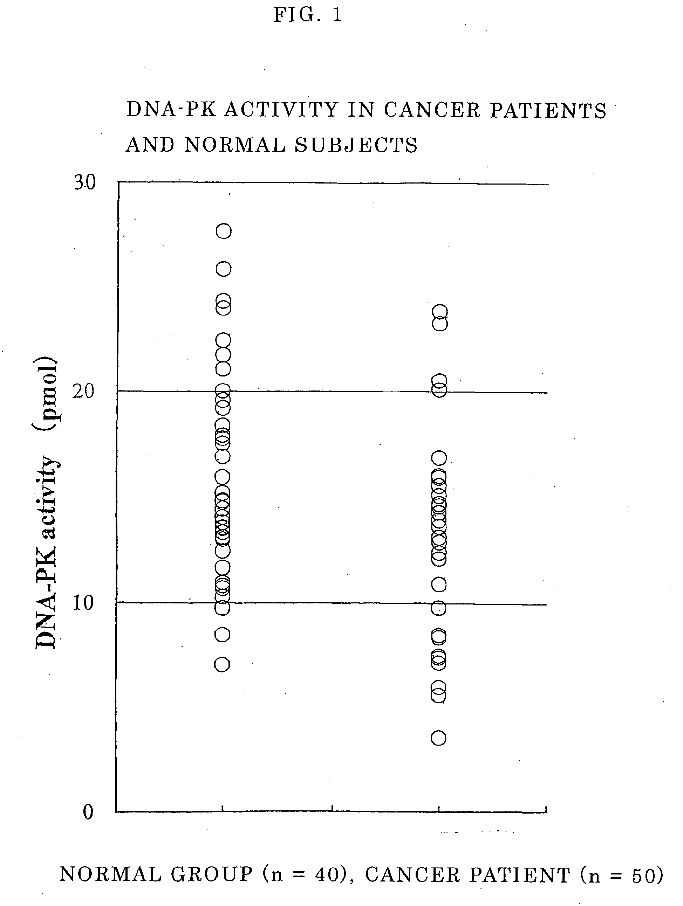

FIG. 1 shows relationship between DNA-dependent protein kinase activity and chromosome abnormality. FIG. 1 is a graph showing the relationship between DNA-dependent protein kinase activity and chromosome abnormality by plotting DNA-dependent protein kinase activity on the horizontal axis and the value of chromosome abnormality on the vertical axis. In FIG. 1, the results of the cancer patients are shown by filled circles and the results of the normal group are shown by open circles. With reference to FIG. 1, it is confirmed that the value of chromosome abnormality decreases with an increase in DNA-dependent protein kinase activity and, reversely, that the value of chromosome abnormality increases with a decrease in DNA-dependent protein kinase activity. Additionally, a tendency is observed that the DNA-dependent protein kinase activity of the cancer patients is lower than that of the normal group. Namely, it is confirmed that cancer susceptibility increases with a decrease in the activity of DNA-dependent protein kinase that is an enzyme playing an important role in the repair process of double-stranded DNA breaks.

### Example 2

DNA-dependent protein kinase activity in lymphoid cells of cancer patients and a normal group was measured. The lymphoid cells of the cancer patients were derived from patients suffering from breast cancer, uterine cancer, head and neck cancer, or malignant lymphoma. DNA-dependent protein kinase activity of lymphoid cells was measured for each of 50 cancer patients and 40 people of the normal group, as in Example 1.

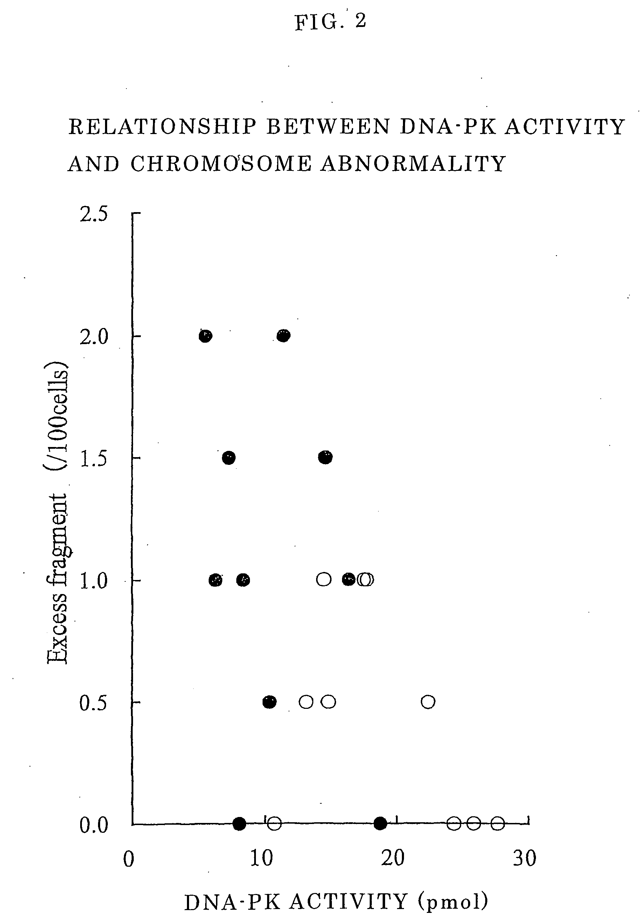

FIG. 2 shows the result. FIG. 2 is a graph showing the measurement results of DNA-dependent protein kinase activity in lymphoid cells of the cancer patients and the normal group. In FIG. 2, DNA-dependent protein kinase activity is plotted on the vertical axis. As shown in FIG. 2, DNA-dependent protein kinase activity in lymphoid cells derived from the cancer patients was significantly lower than that of the normal group. With this result, it is confirmed that cancer diagnosis is possible by measuring DNA-dependent protein kinase activity in lymphoid cells.

As described in detail in the above, the presence of cancerous cells can be investigated by the method for diagnosing cancer according to the present invention, whatever the organ or the cause of the carcinogenesis is. Additionally, a tendency to suffer from cancer can be investigated by the method for determining cancer susceptibility according to the present invention.

Thus, the method for determining cancer susceptibility according to the present invention can investigate not only whether a test subject is actually suffering with cancer but also whether the test subject tends to suffer with cancer. Therefore, the method can be applied to cancer examination.

## Claims

1. A method for diagnosing cancer by measuring DNA-dependent protein kinase activity in cells derived from a test subject.

2. A method for diagnosing cancer, the method comprising the steps of:
measuring DNA-dependent protein kinase activity in cells derived from a test subject;
measuring DNA-dependent protein kinase activity in cells derived from a healthy subject; and
comparing the DNA-dependent protein kinase activity in cells derived from the test subject and the DNA-dependent protein kinase activity in cells derived from the healthy subject.

3. The method for diagnosing cancer according to claim 1 or 2, wherein the cells are lymphoid cells.

4. A cancer diagnosis kit for diagnosing cancer by the method for diagnosing cancer according to any one of claims 1 to 3.

5. The cancer diagnosis kit for diagnosing cancer by the method for diagnosing cancer according to any one of claims 1 to 3, the kit comprising a peptide substrate which is phosphorylated by DNA-dependent protein kinase.

6. A method for determining cancer susceptibility by measuring DNA-dependent protein kinase activity in cells derived from a test subject.

7. A method for determining cancer susceptibility, the method comprising the steps of:
measuring DNA-dependent protein kinase activity in cells derived from a test subject;
measuring DNA-dependent protein kinase activity in cells derived from a healthy subject; and
comparing the DNA-dependent protein kinase activity in cells derived from the test subject and the DNA-dependent protein kinase activity in cells derived from the healthy subject.

8. The method for determining cancer susceptibility according to claim 6 or 7, wherein the cells are lymphoid cells.

9. A cancer susceptibility determination kit for determining cancer susceptibility by the method for determining cancer susceptibility according to any one of claims 6 to 8.

10. The cancer susceptibility determination kit for diagnosing cancer by the method for diagnosing cancer susceptibility according to any one of claims 6 to 9, the kit comprising a peptide substrate which is phosphorylated by DNA-dependent protein kinase.
